# EUROPEAN PATENT APPLICATION

(11) **EP 3 415 507 A1**
(43) Date of publication of application: **19.12.2018**
(21) Application number: 17175754.5
(22) Date of filing: 13.06.2017
(51) Int. Cl.: C07D 285/13, C07C 329/06

(54) **PROCESS FOR THE MANUFACTURE OF ARYL-THIADIAZOLE-ACETAMIDE COMPOUNDS**

(71) Applicant: SOLVAY SA, 1120 Brussels (BE)
(72) Inventor: BRAUN, Max Josef, 30900 Wedemark (DE); JAUNZEMS, Janis, 30559 Hannover (DE); METZ, Francois, 69540 Irigny (FR)
(74) Representative: Mross, Stefan P.M.

(57) **Abstract**

The present invention concerns processes for the manufacture of aryl-thiadiazole-acetamide compounds or precursors thereof and processes for the manufacture of agriculturally or pharmaceutically active compounds comprising the processes for the manufacture of aryl-thiadiazole-acetamide compounds or precursors thereof.

## Description

The present invention concerns processes for the manufacture of aryl-thiadiazole-acetamide compounds or precursors thereof and processes for the manufacture of agriculturally or pharmaceutically active compounds comprising the processes for the manufacture of aryl-thiadiazole-acetamide compounds or precursors thereof.

Aryl-thiadiazole-acetamide compounds or precursors thereof have been recognized to be useful structural moieties in numerous agriculturally or pharmaceutically active compounds, such as, for example Flufenacet, a herbicide. The routes towards compounds aryl-thiadiazole-acetamide usually proceed via coupling of an 2-hydroxyacetamide compound with thiadiazole intermediates. Such a coupling is disclosed, for example, in US5792872. It has been found that it can be advantageous when the formation of the thiadiazole directly on the aryl moiety in the newly developed process can have advantages, such as employing short routes, good to handle starting materials, commercially available starting materials and overall good yields and waste optimization.

In one aspect, the invention this concerns a process for the manufacture of a compound of formula (I) 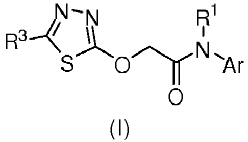
wherein R¹ is selected from the group consisting of H, optionally substituted branched or straight C₁-C₁₂-alkyl, optionally substituted C₃-₈ cycloalkyl, optionally substituted aryl, optionally substituted heterocycloalkyl, and optionally substituted heteroaryl, and wherein Ar is an optionally substituted aryl or heteroaryl group,
wherein R³ is selected from the group consisting of optionally substituted C₁-C₁₂-alkyl, optionally substituted C₃₋₈ cycloalkyl, optionally substituted heterocycloalkyl, and optionally substituted heteroaryl, -CN and -COOR⁴, wherein R⁴ selected from H and a C₁ to C₄ alkyl group
wherein a compound of formula (II), wherein R¹ and Ar are defined as above, 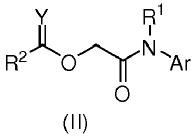
is submitted to at least one cyclization step according to step a), b) or c) wherein when Y in (II) is S, R² is a residue of formula (III) or (V), and when Y in (II) is O, R² is a residue of formula (IV), 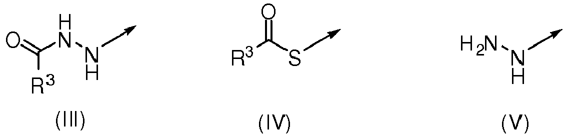 wherein in a) b) and c) are defined as below.

The invention also concerns a process for the manufacture of a compound of formula (II) wherein Y is O and R² is a residue of formula (IV), wherein a compound of formula (VI) is reacted with a compound of formula (VII) and (VIII) 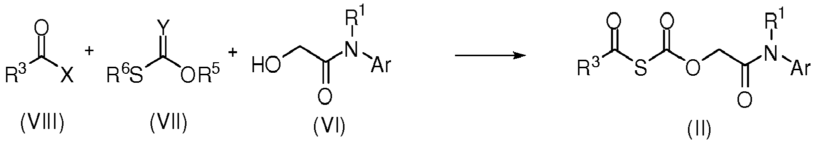
wherein R¹, Ar and R³ are defined as below, X is selected from Cl, Br and F, Y is S or O,
R⁵ is selected from the group consisting of H, optionally substituted branched or straight C₁-C₁₂-alkyl, optionally substituted C₃₋₈ cycloalkyl, optionally C₆ - aryl optionally substituted heterocycloalkyl, and optionally substituted heteroaryl
R⁶ is selected from H and optionally substituted branched or straight C₁-C₁₂-alkyl, or R⁶ is a metal ion to form a thiolate group with the sulphur atom.

Another object of the present invention is a process for the manufacture of a compound of formula (VI), wherein a compound of formula (IX) is reacted with a compound of formula (X) 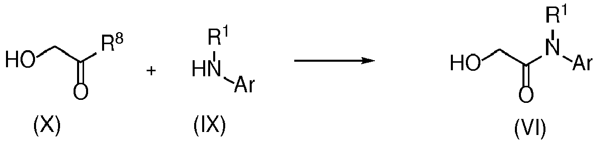 wherein R⁸ is selected from the group consisting of -OR⁷, wherein R is an optionally substituted C₁₋₁₂ alkyl or aryl group, preferably R⁷ is ethyl or methyl, or R⁸ is X, wherein X is selected from the group consisting of Cl, Br and F.

Yet another object of the present invention is a process for the manufacture of a compound of formula (II) wherein Y is S and R² is a residue of formula (III), wherein a compound of formula (II) wherein Y is S and R² is a residue of formula (V) is reacted with a compound of formula (VIII) 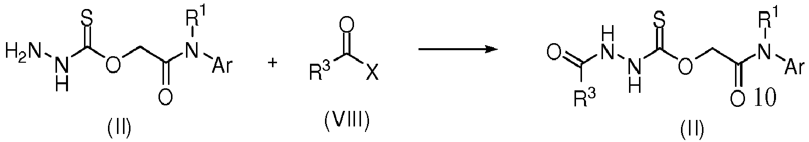 wherein R¹, R³, and X are defined as below.

The invention further concerns a process for the manufacturing of an agriculturally or pharmaceutically active compound or a composition comprising an agriculturally or pharmaceutically active compound, which comprises at least one of the processes according to the present invention , in particular wherein the agriculturally active compound is N-(4-Fluorophenyl)-N-isopropyl-2-{[5-(trifluoromethyl)-1,3,4-thiadiazol-2-yl]oxy}acetamide or a precursor thereof. In the present invention, designations in singular are in intended to include the plural; for example, "a solvent" is intended to denote also "more than one solvent" or "a plurality of solvents".
All aspects and embodiments of the present invention are combinable.

In the context of the present invention, the term "comprising" is intended to include the meaning of "consisting of'.

When a double bond is depicted in a particular E/Z geometry, this is intended to also denote the other geometric form as well as mixtures thereof.

Generally, in the present invention, basic compounds, such as the compound of formula H₂NNH₂ or (XIII), can also be used in their form as a salt, for example in the form of a hydrogen halide salt.

In the context of the present invention, the term "C₁-C₁₂-alkyl" is intended to denote straight or branched alkyl groups having one to twelve carbon atoms. The group comprises, for example, methyl, ethyl, n-propyl, isopropyl, n-, iso-, sec- and t-butyl, n-pentyl and its isomers, n-hexyl and its isomers, 1,3-dimethylbutyl, 3,3-dimethylbutyl, n-heptyl and its isomers, n-octyl and its isomers, n-nonyl and its isomers, n-decyl and its isomers, n-undecyl and its isomers and n-dodecyl and its isomers. Often, the C₁ to C₄ alkyl groups are the most preferred groups of the C₁-C₁₂ alkyl group. The term "C₁-C₄-alkyl" is intended to denote straight or branched alkyl groups having one to four carbon atoms. This group comprises methyl, ethyl, n-propyl, isopropyl, n-, iso-, sec- and t-butyl.

The term "C₃-C₈ cycloalkyl group" intends to denote mono-, bi- or tricyclic hydrocarbon groups comprising 3 to 10 carbon atoms, especially 3 to 6 carbon atoms. Examples of monocyclic groups include cyclopropyl, cyclobutyl, cyclopentyl, cyclohexyl, cycloheptyl or cyclooctyl. Examples of bicyclic groups include bicyclo[2.2.1]heptyl, bicyclo[3.1.1]heptyl, bicyclo[2.2.2]octyl and bicyclo[3.2.1]octyl. Examples of tricyclic groups are adamantyl and homoadamantyl.

The term "aryl group" intends to denote C₅-C₁₈ monocyclic and polycyclic aromatic hydrocarbons with 5 to 18 carbon atoms in the cyclic system. Specifically, this definition comprises, for example, the meanings cyclopentadienyl, phenyl, cycloheptatrienyl, cyclooctatetraenyl, naphthyl and anthracenyl.

In the context of the present invention, the term "heteroaryl" intends to denote C₅-C₁₈ monocyclic and polycyclic aromatic hydrocarbons with 5 to 18 carbon atoms in the cyclic system, wherein one or more methine (-C=) and/or vinylene (-CH=CH-) groups are replaced by trivalent or divalent heteroatoms, in particular nitrogen, oxygen and/or sulphur, respectively, in such a way as to maintain the continuous π-electron system characteristic of aromatic systems. Specifically, this definition comprises, for example, the meanings 2-furyl, 3-furyl, 2-thienyl, 3-thienyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl; 1-pyrrolyl, 1-pyrazolyl, 1,2,4-triazol-1-yl, 1-imidazolyl, 1,2,3-triazol-1-yl, 1,3,4-triazol-1-yl; 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

A C₁-C₄ alkyl group, C₁-C₁₂ alkyl group, C₃-C₈ cycloalkyl group, aryl or heteroaryl group each can be optionally substituted by one or more substituents of the following group consisting of R', -X", -OR', -SR', -NR'₂, -SiR'₃, -COOR', - (C-O)R', -CN and -CONR'₂, wherein R' is selected independently, from the group consisting of hydrogen or C₁-C₁₂-alkyl group and X" is selected from the group consisting of F, Cl, Br, or I.

In its broadest aspect, the invention this concerns a process for the manufacture of a compound of formula (I) 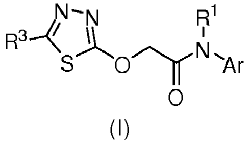
wherein R¹ is selected from the group consisting of H, optionally substituted branched or straight C₁-C₁₂-alkyl, optionally substituted C₃-₈ cycloalkyl, optionally substituted aryl, optionally substituted heterocycloalkyl, and optionally substituted heteroaryl, and wherein Ar is an optionally substituted aryl or heteroaryl group,
wherein R³ is selected from the group consisting of optionally substituted C₁-C₁₂-alkyl, optionally substituted C₃₋₈ cycloalkyl, optionally substituted heterocycloalkyl, and optionally substituted heteroaryl, -CN and -COOR⁴, wherein R⁴ selected from H and a C₁ to C₄ alkyl group wherein a compound of formula (II) wherein R¹ and Ar are defined as above 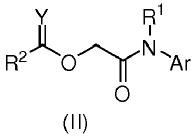
is submitted to at least one cyclization step according to step a), b) or c) wherein when Y in (II) is S, R² is a residue of formula (III) or (V), and when Y in (II) is O, R² is a residue of formula (IV), 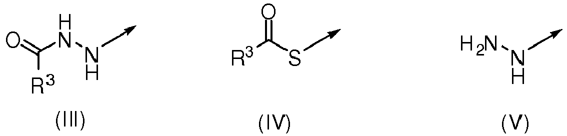 wherein in a) b) and c) are defined as follows:

In step a), the compound of formula (II), wherein Y is S and R² is a residue of formula (III), is cyclized in the presence of a carboxylic acid anhydride, a water removing agent or a base to form the compound of formula (II)

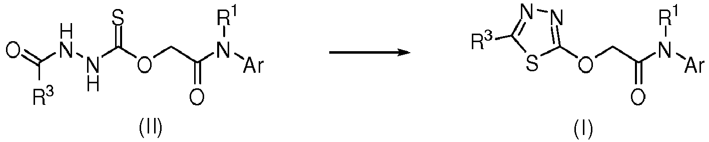

The base that can be present in the cyclization of formula (II), wherein Y is S and R² is a residue of formula (III), can be an organic base, such as for example triethylamine or pyridine, or an inorganic base, such as for example KOH, NaOH, a metal alcoholate such as tBuOK, and LiOH. The preferred base is triethylamine. The carboxylic acid anhydride that can be present in the cyclization of formula II), wherein Y is S and R² is a residue of formula (III) can be an unsubstituted carboxylic acid anhydride, for example acetic acid anhydride, or a substituted carboxylic anhydride, for example trifluoroacetic acid anhydride. Carboxylic acid anhydrides derived from optionally substituted C₁₋₄ carboxylic acids are preferred. The water removing agent that can be present in the cyclization of formula (II), wherein Y is S and R² is a residue of formula (III), can be selected, for example, from the group consisting of sulphuric acid, oleum, toluenesulfonic acid, orthoformates such as triethylorthoformate, zeolites, silica gel, NH₄OAc, molecular sieves and P₂O₅.

In step b), the compound of formula (II), wherein Y is O and R² is a residue of formula (IV) is reacted with hydrazine

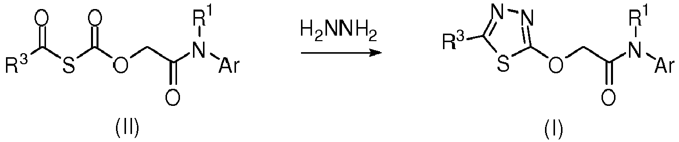

In the reaction of (II) wherein Y is O and R² is a residue of formula (IV) with hydrazine, optionally, a base can be present. "A base", in this context, denotes a base other than H₂NNH₂, which chemically also could be considered to be a base. « A base » can be, for example, an organic base, such as for example triethylamine or pyridine, or an inorganic base, such as for example KOH, tBuOK, NaOH or LiOH. It can be advantageous to convert hydrazine into a hydrazone by reaction with an aldehyde or ketone, such as benzaldehyde or acetone, prior to the reaction with (II), in order to enhance selectivity in the addition to the desired C(O) function in (II). In this aspect, it is advantageous if an acidic catalyst is present when the hydrazone-(II) product is cyclized, for example HCI or Na₂SO₄.

In step c), the compound of formula (II), wherein Y is S and R² is a residue of formula (V) is cyclized in the presence of a carboxylic acid anhydride of formula R³C(O)-O-C(O)R³

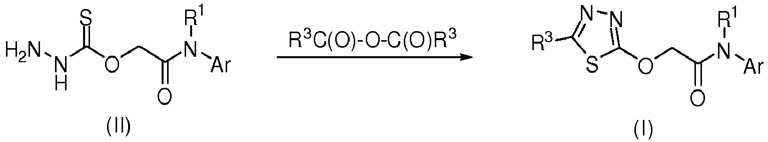

In the cyclization reaction of (II) wherein Y is S and R² is a residue of formula (V) in the presence of a carboxylic acid anhydride of formula R³C(O)-O-C(O)R³, optionally, a base can be present. A suitable base can be, for example, an organic base, such as for example triethylamine or pyridine, or an inorganic base, such as for example KOH, tBuOK, NaOH or LiOH. The preferred carboxylic acid anhydride of formula R³C(O)-O-C(O)R³ is trifluoroacetic acid anhydride.

Another object of the present invention is a process for the manufacture of a compound of formula (II) wherein Y is O and R² is a residue of formula (IV), wherein a compound of formula (VI) is reacted with a compound of formula (VII) and (VIII) 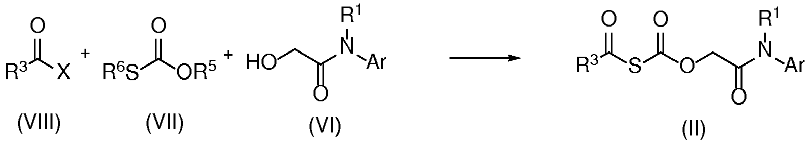
wherein R¹, Ar and R³ are defined as above, X is selected from Cl, Br and F, Y is O,
R⁵ is selected from the group consisting of H, optionally substituted branched or straight C₁-C₁₂-alkyl, optionally substituted C₃-₈ cycloalkyl, optionally C₆ - aryl optionally substituted heterocycloalkyl, and optionally substituted heteroaryl, wherein methyl and ethyl are preferred
R⁶ is selected from H and optionally substituted branched or straight C₁-C₁₂-alkyl, or R⁶ is a metal ion to form a thiolate group with the sulphur atom of (VII), wherein R⁶ preferably is H.

In the process for the manufacture of a compound of formula (II) wherein Y is O and R² is a residue of formula (IV), wherein a compound of formula (VI) is reacted with a compound of formula (VII) and (VIII), optionally a base can be present. A suitable base can be, for example, an organic base, such as for example triethylamine or pyridine, or an inorganic base, such as for example KOH, tBuOK, NaOH or LiOH.

Another object of the present invention is a process for the manufacture of a compound of formula (II) wherein Y is O and R² is a residue of formula (IV), wherein a compound of formula (VI) is reacted with a compound of formula (VII) and (VIII) of a compound of formula (VI), which further comprises a step for the manufacture of a compound of formula (VI), wherein a compound of formula (IX) is reacted with a compound of formula (X) 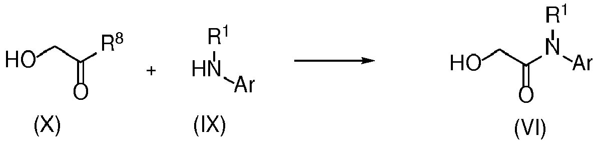 wherein R⁸ is selected from the group consisting of -OR⁷, wherein R is an optionally substituted C₁₋₁₂ alkyl or aryl group, preferably R⁷ is ethyl or methyl, or R⁸ is X, wherein X is selected from the group consisting of Cl, Br and F.

The invention also concerns a process for the manufacture of a compound of formula (II) wherein Y is S and R² is a residue of formula (III), wherein a compound of formula (II) wherein Y is S and R² is a residue of formula (V) is reacted with a compound of formula (VIII) 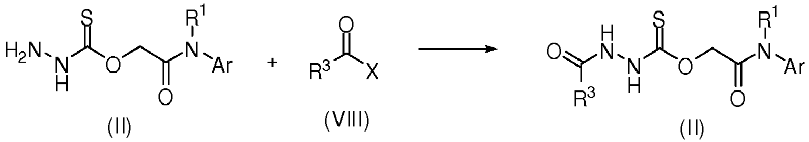 wherein R¹ and R³ are defined as above, and X is selected from the group consisting of Cl, F and Br. In the process for the manufacture of a compound of formula (II) wherein Y is S and R² is a residue of formula (III), wherein a compound of formula (II) wherein Y is S and R² is a residue of formula (V) is reacted with a compound of formula (VIII), optionally a base can be present. A suitable base can be, for example, an organic base, such as for example triethylamine or pyridine, or an inorganic base, such as for example KOH, tBuOK, NaOH or LiOH.

Another object of the present invention is a process for the manufacture of a compound of formula (II) wherein R² is a residue of formula (V), wherein a compound of formula (VI) is treated successively with CS₂, a base, a salt of acetic acid, and hydrazine . The base preferably is KOH. The salt of acetic acid preferably is sodium acetate.

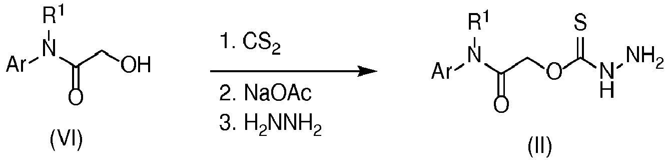

In all aspects and processes according to the present invention, R³ often is selected from a C₁ to C₄ alkyl group, which is preferably halogenated, and more preferably R³ is selected from CF₂H and CF₃. Likewise, in all aspects and processes according to the present invention, R¹ is often selected from a C₁ to C₄ alkyl group, wherein preferably, R¹ is an isopropyl group. Ar preferably is a substituted aryl group, more preferably an optionally substituted phenyl group, even more preferably a halogenated phenyl group, and most preferably a 4-fluorophenyl group.

The invention also concerns a process for the manufacture of a compound of formula (I), wherein a compound of formula (II) is submitted to at least one cyclization step according to step a), b) or c), and which further comprises at least one of the processes for
- the manufacture of a compound of formula (II) wherein Y is O and R² is a residue of formula (IV), wherein a compound of formula (VI) is reacted with a compound of formula (VII) and (VIII), which optionally further comprises a step for the manufacture of a compound of formula (VI), wherein a compound of formula (IX) is reacted with a compound of formula (X)
- the manufacture of a compound of formula (II) wherein Y is S and R² is a residue of formula (III), wherein a compound of formula (II) wherein Y is S and R² is a residue of formula (V) is reacted with a compound of formula (VIII)
- the manufacture of a compound of formula (II) wherein R² is a residue of formula (V), wherein a compound of formula (VI) is treated successively with CS₂, a base, a salt of acetic acid, and hydrazine

Another object according to the present invention is a process for the manufacturing of an agriculturally or pharmaceutically active compound or a composition comprising an agriculturally or pharmaceutically active compound, which comprises at least one of the processes according to the present invention , in particular wherein the agriculturally active compound is N-(4-Fluorophenyl)-N-isopropyl-2-{[5-(trifluoromethyl)-1,3,4-thiadiazol-2-yl]oxy}acetamide or a precursor thereof.

Should the disclosure of any patents, patent applications, and publications which are incorporated herein by reference conflict with the description of the present application to the extent that it may render a term unclear, the present description shall take precedence.
The following examples are intended to further explain the invention without limiting it.

The starting materials for the processes and steps according to the present invention can be obtained from commercial sources or can be manufactured according to procedures known from the public domain. The manufacture of compounds of formula (VI) is described, for example, in EP0060426.

### Example 1: 2,2,2-trifluoroacetic (2-((4-fluorophenyl)(isopropyl)amino)-2-oxoethyl carbonic) thioanhydride

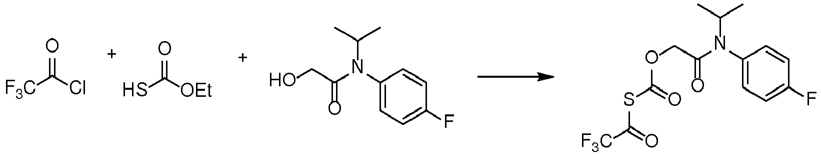

13.25 g trifluoroacetic acid chloride (0.1 mol) are added to a mixture of 15,9 g (0.15 mol) O-ethyl S-hydrogen carbonothioate and 1 g pyridine in 50 mL. The product, trifluoroacetyl ethoxycarbonyl sulphide, can be used in the following step as a crude product, or can be distilled to obtain a purer intermediary product. 10 g (0.1 mol) of the intermediary trifluoroacetyl ethoxycarbonyl sulphide is subsequently treated with N-(4-fluorophenyl)-2-hydroxy-N-isopropylacetamide in the presence of triethylamine. The 2,2,2-trifluoroacetic (2-((4-fluorophenyl)(isopropyl)amino)-2-oxoethyl carbonic) thioanhydride can be used in the next step as a crude product.

### Example 2: N-(4-fluorophenyl)-N-isopropyl-2-((5-(trifluoromethyl)-1,3,4-thiadiazol-2-yl)oxy)acetamide

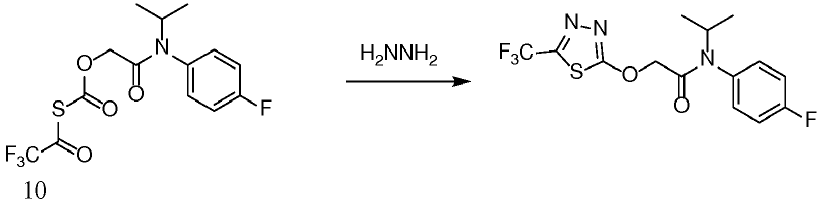

3.7 g (0.01 mol) 2,2,2-trifluoroacetic (2-((4-fluorophenyl)(isopropyl)-amino)-2-oxoethyl carbonic) thioanhydride is treated with 0.01 mol hydrazine hydrochloride in dichloromethane. Flufenacet is obtained after workup, such as recrystallization, in good yields and purity.

## Claims

1. Process for the manufacture of a compound of formula (I) 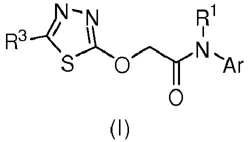
wherein R¹ is selected from the group consisting of H, optionally substituted branched or straight C₁-C₁₂-alkyl, optionally substituted C₃-₈ cycloalkyl, optionally substituted aryl, optionally substituted heterocycloalkyl, and optionally substituted heteroaryl, and wherein Ar is an optionally substituted aryl or heteroaryl group,
wherein R³ is selected from the group consisting of optionally substituted C₁-C₁₂-alkyl, optionally substituted C₃-₈ cycloalkyl, optionally substituted heterocycloalkyl, and optionally substituted heteroaryl, -CN and -COOR⁴, wherein R⁴ selected from H and a C₁ to C₄ alkyl group
wherein a compound of formula (II) wherein R¹ and Ar are defined as above 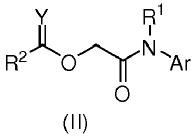
is submitted to at least one cyclization step according to step a), b) or c) wherein when Y in (II) is S, R² is a residue of formula (III) or (V), and when Y in (II) is O, R² is a residue of formula (IV), 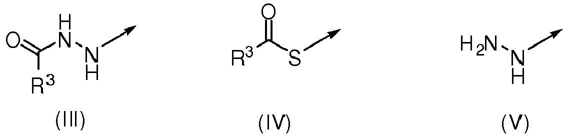 wherein in
step a), the compound of formula (II), wherein Y is S and R² is a residue of formula (III) is cyclized in the presence of a carboxylic acid anhydride, a water removing agent or a base to form the compound of formula (II) 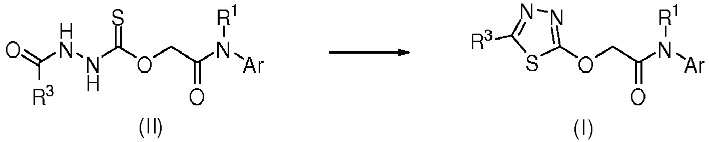 or wherein in
step b), the compound of formula (II), wherein Y is O and R² is a residue of formula (IV) is reacted with hydrazine 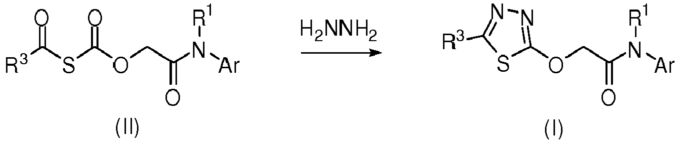 or wherein in
step c), the compound of formula (II), wherein Y is S and R² is a residue of formula (V) is cyclized in the presence of a carboxylic acid anhydride of formula R³C(O)-O-C(O)R³

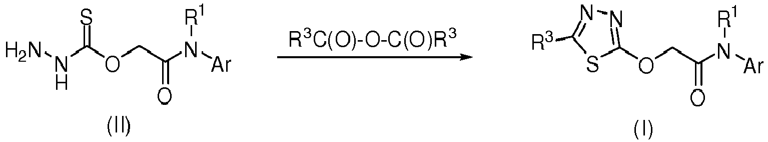

2. Process according to claim 1, wherein R³ is selected from a C₁ to C₄ alkyl group, which is preferably halogenated.

3. Process according to claim 2, wherein R³ is selected from CF₂H and CF₃.

4. Process according to anyone of claims 1 to 3, wherein R¹ is selected from a C₁ to C₄ alkyl group.

5. Process according to claim 4, wherein R¹ is an isopropyl group.

6. Process according to anyone of claims 1 to 5, wherein in step c), the carboxylic acid anhydride is trifluoroacetic acid anhyride.

7. Process according to anyone of claims 1 to 5, wherein in step a), the base is triethylamine.

8. Process according to anyone of claims 1 to 7, wherein Ar is a substituted aryl group, more preferably a 4-fluoro-phenylgroup.

9. Process for the manufacture of a compound of formula (II) wherein Y is O and R² is a residue of formula (IV), wherein a compound of formula (VI) is reacted with a compound of formula (VII) and (VIII) 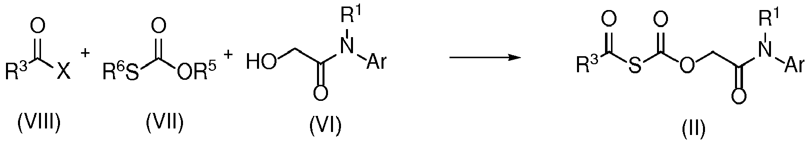
wherein R¹, Ar and R³ are defined as in any one of the preceding claims, X is selected from Cl, Br and F, Y is S or O,
R⁵ is selected from the group consisting of H, optionally substituted branched or straight C₁-C₁₂-alkyl, optionally substituted C₃-₈ cycloalkyl, optionally C₆ - aryl optionally substituted heterocycloalkyl, and optionally substituted heteroaryl
R⁶ is selected from H and optionally substituted branched or straight C₁-C₁₂-alkyl, or R⁶ is a metal ion to form a thiolate group.

10. Process according to claim 9, which further comprises a step for the manufacture of a compound of formula (VI), wherein a compound of formula (IX) is reacted with a compound of formula (X) 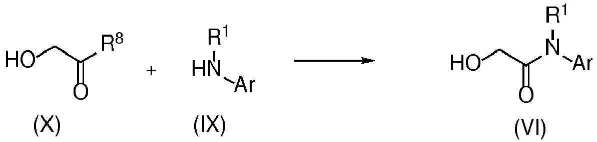 wherein R⁸ is selected from the group consisting of -OR⁷, wherein R is an optionally substituted C₁₋₁₂ alkyl or aryl group, preferably R⁷ is ethyl or methyl, or R⁸ is X, wherein X is selected from the group consisting of Cl, Br and F.

11. Process for the manufacture of a compound of formula (II) wherein Y is S and R² is a residue of formula (III), wherein a compound of formula (II) wherein Y is S and R² is a residue of formula (V) is reacted with a compound of formula (VIII) 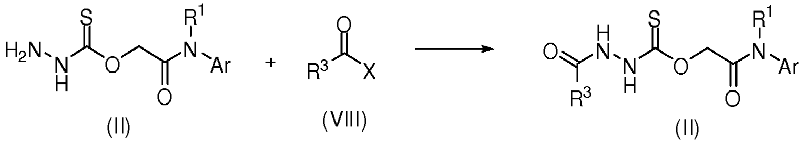 wherein R¹, R³, and X are defined according to any of the preceding claims.

12. Process for the manufacture of a compound of formula (II) wherein R² is a residue of formula (V), wherein a compound of formula (VI) is treated successively with CS₂, a base, a salt of acetic acid, and hydrazine.

13. Process for the manufacture of a compound of formula (I), which comprises at least one of the processes of claim 9 to 12.

14. Process for the manufacturing of an agriculturally or pharmaceutically active compound or a composition comprising an agriculturally or pharmaceutically active compound, which comprises at least one of the processes according to anyone of claims 1 to 13.

15. Process according to claim 14, wherein the agriculturally active compound is N-(4-Fluorophenyl)-N-isopropyl-2-{[5-(trifluoromethyl)-1,3,4-thiadiazol-2-yl]oxy}acetamide or a precursor thereof.
